# EUROPEAN PATENT SPECIFICATION

(11) **EP 3 155 952 B1**
(45) Date of publication and mention of the grant of the patent: **02.10.2019**
(21) Application number: 15806899.9
(22) Date of filing: 01.05.2015
(51) Int. Cl.: A61B 1/00, A61B 8/12, A61B 10/02, A61B 17/34, A61B 10/04

(54) **BIOPSY SYSTEM**
BIOPSIESYSTEM
SYSTÈME DE BIOPSIE

(30) Priority: 10.06.2014 JP 2014119922
(43) Date of publication of application: 19.04.2017
(73) Proprietor: Olympus Corporation, Tokyo 192-8507 (JP)
(72) Inventor: UEMICHI, Katsuji, Hachioji-shi Tokyo 192-8507 (JP); SAKAMOTO, Yuji, Hachioji-shi Tokyo 192-8507 (JP)
(74) Representative: Schicker, Silvia
(86) International application number: PCT/JP2015/063104
(87) International publication number: WO 2015/190188

(56) References cited:
- JP-A- H05 253 178
- JP-A- H11 128 233
- JP-A- H11 244 223
- JP-A- 2012 161 591
- US-A- 6 149 598
- US-A1- 2014 094 658

## Description

### Technical Field

The present invention relates to a biopsy system.

Priority is claimed on Japanese Patent Application No. 2014-119922, filed June 10, 2014.

### Background Art

In the related art, a so-called "biopsy" examination technique, in which a tiny amount of body tissues is sampled, and the sample is observed using a microscope, is known in the art. When tissues in a deep part of organs and the like are sampled, it is difficult to perform observation using an optical endoscope. Therefore, in some cases, an ultrasonogram of an organ is captured using an ultrasonic endoscope and the like, and a biopsy needle is inserted into the organ under the ultrasonic observation to sample the tissue.

In Patent Literature 1, a treatment tool for biopsy used for this purpose is disclosed. This treatment tool is provided with a tubular needle tube having a sharp point on the end. As the needle tube is inserted into the tissues, part of the tissues is entered into the inside of the needle tube. By removing the needle tube, it is possible to
sample the tissues entered into the inside.

In Patent Literature 2, an endoscopic treatment tool having a flexible guide tube and a flexible reinforcing tube into which the flexible guide tube is inserted is disclosed. In this endoscopic treatment tool, the flexible reinforcing tube protects the flexible guide tube so as to prevent damage to the flexible guide tube.

Patent Literature 3 discloses an ultrasound endoscope comprising a head grip assembly and an elongated insertion instrument, the insertion instrument having a flexible section, an angle section or flexible joint, and a rigid distal end section. The insertion instrument further comprises an endoscopic image pickup portion and an ultrasound scanner. An instrument passage is provided in a distal end casing between the endoscopic image pickup portion and the ultrasound scanner. A rigid instrument passage pipe, fitted in the instrument passage, acts as a link member for connecting a biopsy channel (i.e., a flexible tube) with the instrument passage. A puncture instrument, placed into the ultrasound endoscope in a preparatory stage or before introducing the endoscopic insertion instrument into a body cavity of a patient, comprises a flexible sleeve, a sharp-pointed puncture needle, and a flexible narrow tube that is connected to the proximal end of the puncture needle.

Patent Literature 4 discloses a medical system including an endoscope and a guide sheath, the endoscope having an insertion unit and a manipulation unit. An introduction channel, in which the guide sheath can be inserted, is formed in the endoscope. The guide sheath is used as a guide for guiding a distal end of a medical device to a target area in a body and has a bent section and a support section, each formed in a tubular shape. An opening section through which a medical device can protrude and retract is formed at the most distal side of a soft section of the bent section. The medical device may be a biopsy forceps that is inserted into the guide sheath from the proximal end of the guide sheath to perform appropriate treatment of a target area.

Patent Literature 5 discloses an ultrasonic endoscope including an elongated flexible insertion portion having a distal end rigid portion, a bendable curved portion connected to the rear end of the distal end rigid portion, an elongated portion and a flexible portion. A treatment instrument insertion opening is formed in the insertion portion and communicates with a treatment instrument channel having a treatment instrument lead-out port. A tissue puncture needle, configured to be guided into a body cavity through the treatment instrument channel, includes an elongated hollow needle, a hollow tube through which the needle is inserted, a grip portion formed of an insulating member, a slider that is provided at the rear end of the grip portion and is fixed to the needle, and a suction cylinder that is detachably attached to the slider.

### Citation List

### Patent Literature

Patent Literature 1: Japanese Unexamined Patent Application, First Publication No. 2001-037765
Patent Literature 2: Japanese Unexamined Patent Application, First Publication No. H10-229987
Patent Literature 3: U.S. Patent Application Publication No. 6 149 598 A
Patent Literature 4: U.S. Patent Application Publication No. 2014/094658 A1
Patent Literature 5: Japanese Patent Application Publication No. H11 128233 A

### Summary of Invention

### Technical Problem

In the biopsy system, the needle tube is inserted into a biopsy target tissue. In some cases, the needle tube may be guided to a proper needling position by making a bending motion in a bending section of a flexible endoscope provided with the bending section capable of being bent.

The needle tube used along with the endoscope has a sharp distal end portion for proper needling to the tissue. In order to prevent damage to the inside of the endoscope,
the needle tube is surrounded by a tubular flexible sheath and is guided to the needling target tissue through the inside of the endoscope.

The flexible sheath guided to the needling target portion through the inside of the endoscope may be elastically deformed by an external force from the endoscope in the course of guidance. For this reason, a relative positional relationship between the flexible sheath and the needle tube may change as the flexible sheath expands or contracts.

In order to reduce a variation in the relative positional relationship between the needle tube and the flexible sheath, forming the flexible sheath to be as hard as the needle tube may be considered. However, in this case, when the hard flexible sheath is disposed inside the bending section of the endoscope, the bending section may not be easily bent.

In view of the aforementioned problems, an object of the present invention is to provide a biopsy system capable of suppressing a flexible sheath from easily expanding or contracting as a whole and preventing degradation of bendability of a bending section of an endoscope.

### Solution to Problem

According to a first aspect of the present invention, a biopsy system includes the features of claim 1. A biopsy system may include: an endoscope having an insertion portion provided with a channel and inserted into a living body and an active bending section disposed in part of the insertion portion and configured to cause part of the channel to actively bend; and an endoscopic treatment tool capable of being inserted into the channel. The endoscopic treatment tool has: a tubular flexible sheath capable of being inserted into the channel; a needle tube provided with a sharp end portion capable of puncturing a tissue for biopsy and disposed inside the sheath; and a manipulation portion fixed to the sheath and moving the needle tube inside the sheath. The sheath has: a distal tube portion disposed in a distal portion of the sheath including a distal end of the sheath; a proximal tube portion fixed to a proximal end of the distal tube portion and aligned along the same axial line as that of the distal tube portion, the proximal tube portion having flexibility lower than that of the distal tube portion; and a connecting portion configured to connect the distal tube portion and the proximal tube portion. A boundary portion between the distal tube portion and the proximal tube portion is positioned in a proximal side relative to a proximal end of the active bending section in a positional relationship in which the distal end of the sheath protrudes from a distal end of the channel and is optically observable by the endoscope.

According to a second aspect of the present invention, in the biopsy system according to the first aspect, the connecting portion may be a tubular member in which the proximal end of the distal tube portion and a distal end of the proximal tube portion are internally placed together. The distal tube portion may have: a flexible coil fixed to the connecting portion; and a cover attached to the coil such that the cover covers an outer circumferential surface of the coil. A difference between an outer diameter of the cover and an outer diameter of the connecting portion may be smaller than a difference between an outer diameter of the proximal tube portion and the outer diameter of the connecting portion.

According to a third aspect of the present invention, in the biopsy system according to the second aspect, an elastic deformation amount of the distal tube portion per unit length in a center line direction caused by a self weight of the distal tube portion may be larger than an elastic deformation amount of the proximal tube portion per unit length in the center line direction caused by the self weight of the distal tube portion.

According to a fourth aspect of the present invention, in the biopsy system according to the third aspect, an outer circumferential surface of the cover may have an uneven shape to follow the outer circumferential surface of the coil.

According to a fifth aspect of the present invention, in the biopsy system according to the fourth aspect, the endoscope may have: an imaging unit capable of imaging the endoscopic treatment tool and a biopsy target portion; and an ultrasonic scanning mechanism disposed in a distal side relative to the imaging unit. The channel may have: a slope portion sloped with respect to a center line of the insertion portion such that sheath is bent toward a scanning range of the ultrasonic scanning mechanism; a channel tube arranged in parallel with the center line of the insertion portion; and an angle portion configured to link the slope portion and the channel tube. In the positional relationship in which the distal end of the sheath protrudes from the distal end of the channel and is optically observable by the endoscope, the end portion of the needle tube may be placed in a distal end side relative to the active bending section and in a proximal side relative to the slope portion when the end portion of the needle tube is maximally shifted to the proximal side by the manipulation portion.

### Advantageous Effects of Invention

According to the present invention, it is possible to provide a biopsy system capable of suppressing a flexible sheath from easily expanding or contracting as a whole and preventing degradation of bendability of a bending section of an endoscope.

### Brief Description of Drawings

Fig. 1 is a schematic diagram illustrating an entire configuration of a biopsy system provided with an endoscopic treatment tool according to an embodiment of the invention.
Fig. 2 is a cross-sectional view illustrating a distal end portion of an ultrasonic endoscope which is an endoscope of the biopsy system according to the embodiment of the invention.
Fig. 3 is a cross-sectional view illustrating a distal end portion of the endoscopic treatment tool.
Fig. 4 is a cross-sectional view illustrating a state in which the endoscopic treatment tool is attached to the ultrasonic endoscope.
Fig. 5 is a partial cross-sectional view illustrating the endoscopic treatment tool.
Fig. 6 is a schematic diagram illustrating a manipulation portion of the endoscopic treatment tool.
Fig. 7 is a perspective view illustrating an attachment state of the endoscopic treatment tool and the ultrasonic endoscope.
Fig. 8 is a schematic diagram for describing operations of the biopsy system.
Fig. 9 is a schematic diagram for describing operations of the biopsy system.
Fig. 10 is a schematic diagram for describing operations of the biopsy system.

### Description of Embodiments

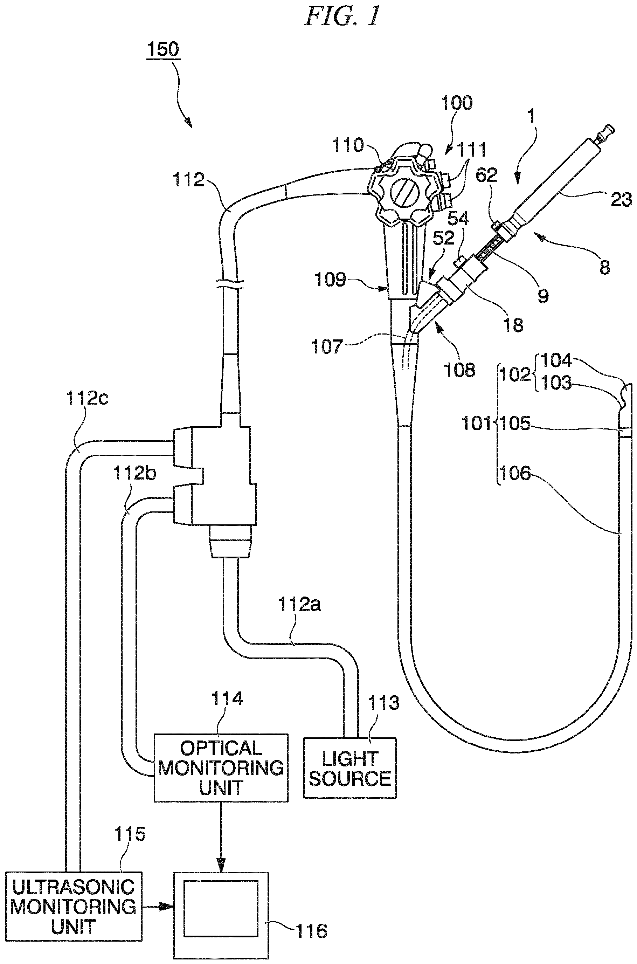

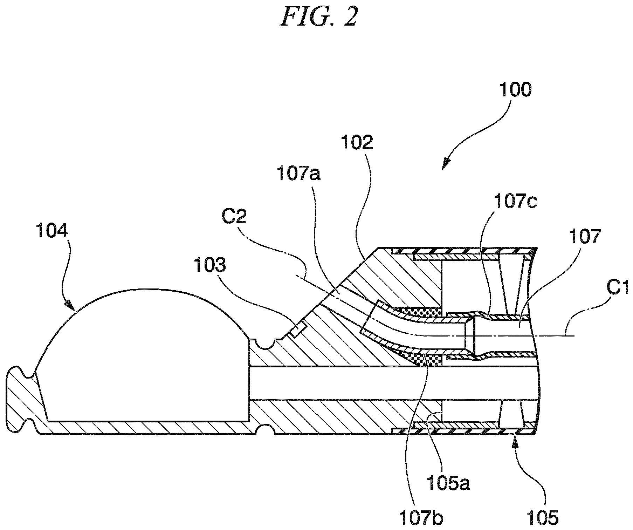

An embodiment of the present invention will now be described. Fig. 1 is a diagram illustrating a schematic configuration of a biopsy system 150 according to this embodiment provided with a treatment tool 1 and an ultrasonic endoscope 100. Fig. 2 is a cross-sectional view illustrating a distal end portion of the ultrasonic endoscope 100 which is an endoscope of the biopsy system 150.

The biopsy system 150 according to this embodiment is a medical instrument that can be used in biopsy for sampling tissues in a living body. The biopsy system 150 includes an ultrasonic endoscope 100 and an endoscopic treatment tool 1 (hereinafter, simply referred to as a "treatment tool").

As illustrated in Fig. 1, the ultrasonic endoscope 100 includes an insertion portion 101 inserted into the living body starting from its distal end, a manipulation unit 109 installed at a proximal end of the insertion portion 101, a universal cord 112 having one end connected to a lateral side of the manipulation unit 109, a light source 113 connected to the other end of the universal cord 112 through a branching cable 112a, an optical monitoring unit 114 connected to the other end of the universal cord 112 through a branching cable 112b, and an ultrasonic monitoring unit 115 connected to the other end of the universal cord 112 through a branching cable 112c.

The insertion portion 101 is provided with a distal end hard section 102, a bending section 105, and a flexible tube section 106 arranged side by side in that order from a distal end side.

The distal end hard section 102 has an optical imaging mechanism (imaging unit) 103 for performing optical observation and an ultrasonic scanning mechanism 104 for performing ultrasonic observation.

The optical imaging mechanism 103 has an imaging optical system having a view field directed diagonally forward from the distal end hard section 102, an image sensor such as a charge-coupled device (CCD) or a complementary metal-oxide semiconductor (CMOS) for detecting a subject image incident through the imaging optical system, and various configurations such as a central processing unit (CPU, not shown) for controlling the operation of the image sensor.

The ultrasonic scanning mechanism (probe) 104 has an ultrasonic wave transceiver (not shown) that emits ultrasonic waves and receives reflected waves. In the ultrasonic scanning mechanism 104, the ultrasonic wave transceiver emits ultrasonic waves, and the waves collide with a scanning target and are reflected therefrom. The reflected waves are received by the ultrasonic wave transceiver, and the signal based on the ultrasonic waves received by the ultrasonic wave transceiver is output to the ultrasonic monitoring unit 115. The ultrasonic scanning mechanism 104 according to this embodiment is used to capture both an ultrasonic image for the biopsy target tissues and an ultrasonic image for the needle tube 3 in the course of a biopsy procedure.

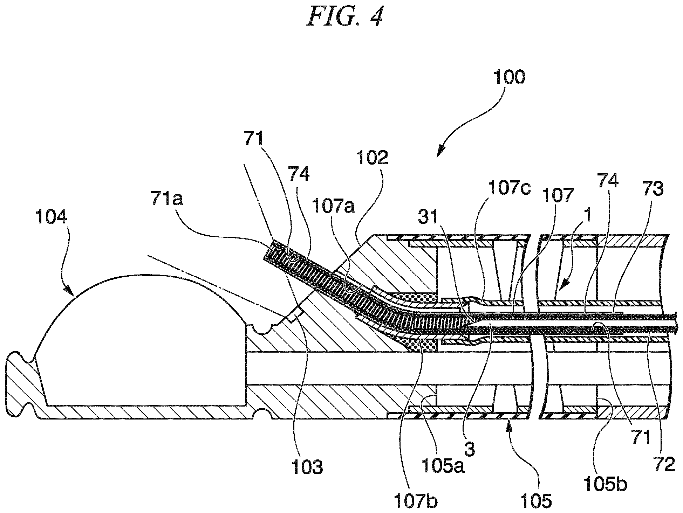

The bending section 105 is formed in a tubular shape, and is an active bending section that can be bent in a predetermined direction by pulling an angle wire (not shown) that is fixed to a distal end 105a of the bending section 105 (refer to Fig. 4) and extends to the manipulation unit 109 using the manipulation unit 109. The bending section 105 according to this embodiment is bendable in two directions along an ultrasonic scanning direction.

According to this embodiment, for example, for treatment of respiratory organs, an endoscope that has a small outer diameter of the insertion portion and is bendable in two directions is used. For example, when digestive organs are treated, an endoscope that has a large outer diameter, has a high degree of freedom of manipulation, and is bendable in four directions may be used.

The flexible tube section 106 is a flexible tubular member formed to guide the distal end hard section 102 to a desired position inside luminal tissues or living body cavities. Each of the bending section 105 and the flexible tube section 106 is internally provided with a channel 107 and a conduit (not shown) for air and water supply or suction.

The channel 107 illustrated in Figs. 1 and 2 is a tubular portion for inserting the treatment tool 1. One end of the channel 107 is opened in the vicinity of the distal end portion of the distal end hard section 102, and the other end is opened to the side face of the distal end side of the manipulation unit 109. A flange-like proximal end port 108 is fixed to the other end of the channel 107. The treatment tool 1 used along with the ultrasonic endoscope 100 can be fixed to the proximal end port 108.

As illustrated in Fig. 2, the channel 107 has a slope portion 107a sloped with respect to an axial line C1 of the insertion portion 101 inside the distal end hard section 102, an angle tube 107b (angle portion) connected to a proximal end of the slope portion 107a, and a channel tube 107c connected to a proximal end of the angle tube 107b.

The slope portion 107a is provided in the distal end hard section 102 by forming a through-hole having a straight line sloped with respect to the axial line C1 of the insertion portion 101 as a center line in the distal end hard section 102. The center line C2 of the through-hole formed in the slope portion 107a is located at a position included in a scanning surface of the ultrasonic scanning mechanism 104. For this reason, when the treatment tool 1 is inserted into the slope portion 107a, the slope portion 107a can guide the needle tube 3 of the treatment tool 1 toward the scanning surface described above. A slope angle of the center line C2 of the slope portion 107a with respect to the axial line C1 of the insertion portion 101 may be set appropriately depending on a treatment target area. According to this embodiment, the center line C2 of the slope portion 107a is inclined, for example, at an angle of 26° with respect to the axial line C1 of the insertion portion 101.

The angle tube 107b is a tube curved or bent at a predetermined angle in order to change a direction of the distal end of the treatment tool 1 guided to the slope portion 107a from the channel tube 107c to follow a direction of the center line C2 of the slope portion 107a. The angle tube 107b links the slope portion 107a and the channel tube 107c. According to this embodiment, the angle tube 107b has an arch shape bent at a certain curvature.

The channel tube 107c is opened to the distal end side of the insertion portion 101 in parallel with the axial line C1 of the insertion portion 101, in the vicinity of the proximal end of the distal end hard section 102, extends substantially in parallel with the axial line C1 of the insertion portion 101 toward the proximal end side of the insertion portion 101, and is fixed to the proximal end port 108.

The manipulation unit 109 illustrated in Fig. 1 has an external surface formed to allow an operator who uses the ultrasonic endoscope 100 to grip and control it. The manipulation unit 109 has a bending manipulation mechanism 110 for pulling the angle wire to bend the bending section 105 and a plurality of switches 111 for supplying air or water, or suction through the conduit.

The light source 113 is a device for emitting illumination light for imaging of the optical imaging mechanism 103.

The optical monitoring unit 114 is configured to display an image captured by the image sensor of the optical imaging mechanism 103 on a display 116.

The ultrasonic monitoring unit 115 is configured to receive the signal output from the ultrasonic scanning mechanism 104 and create and display an image based on this signal on the display 116.

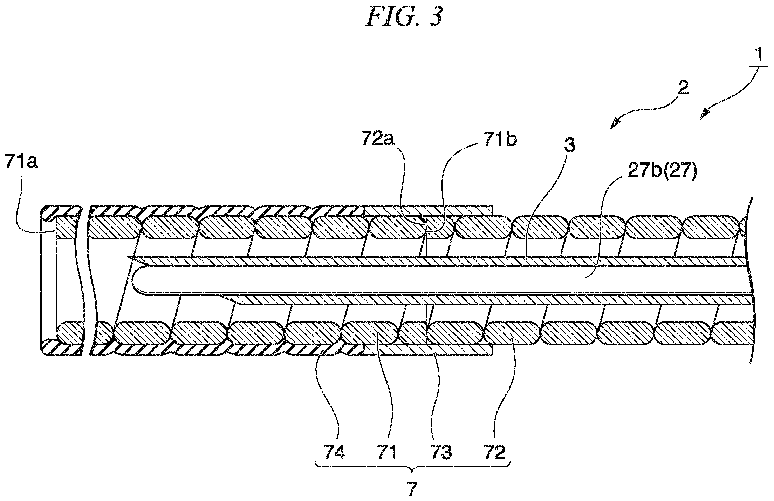

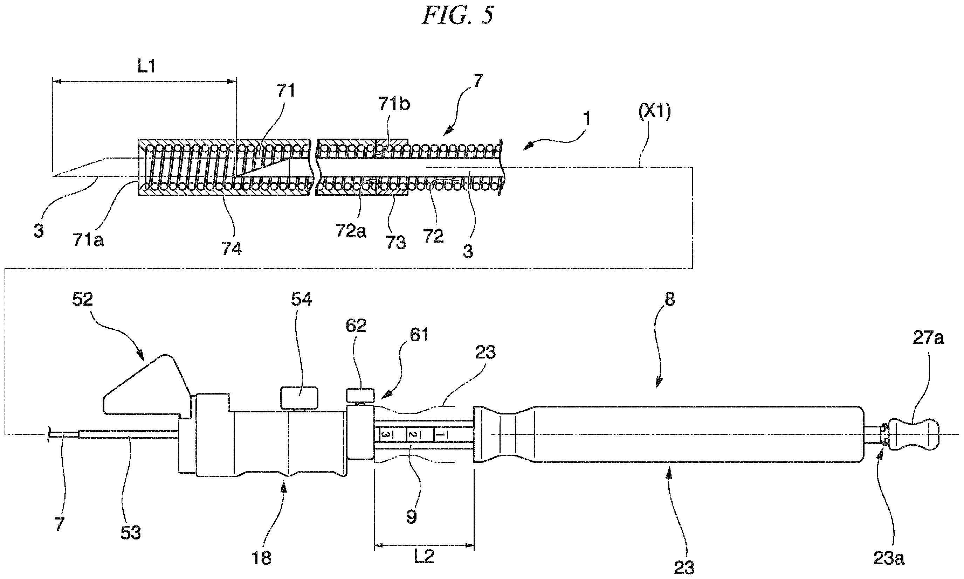

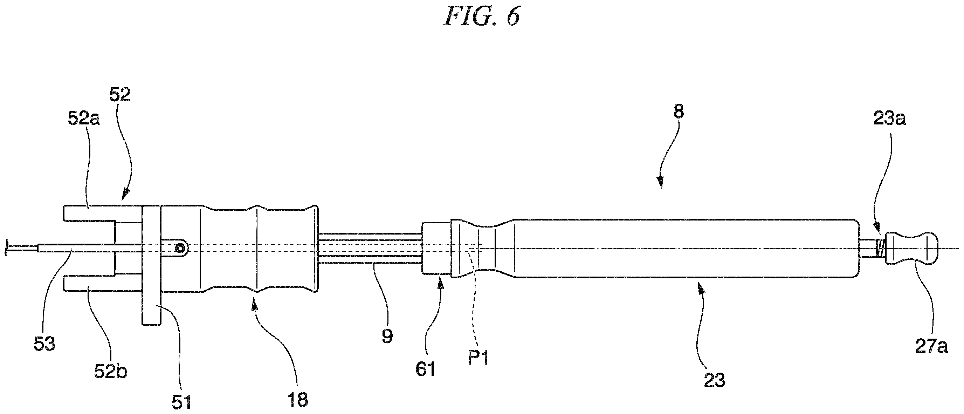

Next, a configuration of the treatment tool 1 will be described. Fig. 3 is a cross-sectional view illustrating a distal end portion of the treatment tool 1. Fig. 4 is a cross-sectional view illustrating a state in which the treatment tool 1 is attached to the ultrasonic endoscope 100. Fig. 5 is a partial cross-sectional view illustrating the treatment tool 1. Fig. 6 is a diagram illustrating a manipulation portion 8 of the treatment tool 1.

As illustrated in Figs. 1 and 3, the treatment tool 1 includes an insertion body 2 inserted into a living body, a manipulation portion (treatment tool manipulation portion) 8 for manipulating the insertion body 2, and a stylet (core metal) 27.

The insertion body 2 is a long member capable of being attached to the channel 107 so as to protrude from the distal end of the insertion portion 101 of the ultrasonic endoscope 100. The insertion body 2 has a needle tube 3 and a tubular sheath 7 into which the needle tube 3 is inserted.

The needle tube 3 is a tubular member that has a distal end and a proximal end and is manipulated to advance or retract using the manipulation portion 8. The needle tube 3 is preferably formed of a flexible and resilient material that can be bent by an external force and can be easily recovered to a straight shape. For example, as a material of the needle tube 3, an alloy material such as a stainless steel alloy, a nickel-titanium alloy, or a cobalt-chromium alloy can be employed.

The needle tube 3 has a sharp distal end to allow needling to the tissues and an opening 31 for suctioning the tissues inside the needle tube 3 formed at the distal end. The opening 31 provided in the distal end of the needle tube 3 is cut to be beveled such that the distal end of the tubular member constituting the needle tube is tilted to the axial line X1 of itself and is formed sharply so as to be inserted into tissues of living organisms. A specific shape of the opening 31 may be selected from various shapes known in the art in consideration of target tissues and the like.

As illustrated in Fig. 3, the sheath 7 has a distal tube portion 71, a proximal tube portion 72, a connecting portion 73, and a covering portion (cover) 74.

The distal tube portion 71 is a flexible tubular member. The distal tube portion 71 is formed in a tubular shape by winding strands made of, for example, metal or the like in a coil shape. The strands of the distal tube portion 71 may be selected appropriately depending on bendability or resilience of the distal tube portion 71. For example, as a material of the strands constituting the distal tube portion 71, a shape memory alloy, a super-elastic alloy, or the like may be employed. The strands constituting the distal tube portion 71 may have a circular cross-sectional shape, a rectangular cross-sectional shape, or the like. The distal end 71a of the distal tube portion 71 is positioned to slightly enter the inside of the covering portion 74. The proximal end 71b of the distal tube portion 71 is fixed to the connecting portion 73.

The proximal tube portion 72 extends from a distal end of the manipulation portion 8. The proximal tube portion 72 is formed of a flexible tubular member. The proximal tube portion 72 has flexibility lower than that of the distal tube portion 71. In other words, the proximal tube portion 72 has rigidity higher than that of the distal tube portion 71. The proximal tube portion 72 is formed in a tubular shape by winding strands made of, for example, metal or the like in a coil shape. The strands constituting the proximal tube portion 72 may be selected appropriately depending on bendability or resilience of the proximal tube portion 72. For example, as a material of the strands constituting the proximal tube portion 72, a shape memory alloy, a super-elastic alloy, or the like may be employed. The strands constituting the proximal tube portion 72 may have a circular cross-sectional shape, a rectangular cross-sectional shape, or the like. The distal end 72a of the proximal tube portion 72 is fixed to the connecting portion 73. The proximal end of the proximal tube portion 72 is fixed to the manipulation portion 8.

Bendability and resilience of the distal tube portion 71 and the proximal tube portion 72 will be described. The distal tube portion 71 is a member having spring properties such that a center line of the distal tube portion 71 is curved due to its own weight when part of the distal tube portion 71 is fixed, and its center line is maintained substantially horizontally. Specifically, the distal tube portion 71 has elasticity set such that, when a length in the center line direction of the distal tube portion 71 is set to a predetermined reference length (300 mm), and one end of the center line direction is cantilevered horizontally, an elastic deformation amount (drooping amount) of the other end is within a range of 100 mm.

The stiffness of the distal tube portion 71 may be set in consideration of the fact that the bending section 105 can be bent across the entire movable range of the bending section 105 of the ultrasonic endoscope 100 while the treatment tool 1 is attached to the ultrasonic endoscope 100. In this case, the stiffness may be set also in consideration of the stiffness of the needle tube 3. Note that it is not necessary that the bending section 105 of the ultrasonic endoscope 100 be able to be bent across its entire movable range at all times.

The proximal tube portion 72 is a member having spring properties such that a center line of the proximal tube portion 72 is bent due to its own weight when part of the proximal tube portion 72 is fixed, and its center line is maintained substantially horizontally. Specifically, the proximal tube portion 72 has elasticity set such that, when a length in the center line direction of the proximal tube portion 72 is set to a predetermined reference length (300 mm), and one end of the center line direction is cantilevered horizontally, an elastic deformation amount (drooping amount) of the other end is within a range of 50 mm.

Focusing on a relationship of the drooping amount (hereinafter referred to as a "drooping ratio") caused by a self weight per unit length between the distal tube portion 71 and the proximal tube portion 72, the drooping ratio of the distal tube portion 71 is higher than that of the proximal tube portion 72. For example, the distal tube portion 71 has a drooping ratio of 0.333 (the drooping amount was 100 mm with respect to the reference length), and the proximal tube portion 72 has a drooping ratio of 0.167 (the drooping amount was 50 mm with respect to the reference length). Preferably, the drooping ratio of the distal tube portion 71 is set to 1.5 to 2.5 times that of the proximal tube portion 72.

The connecting portion 73 is a member for coupling the distal tube portion 71 and the proximal tube portion 72 with each other. The connecting portion 73 according to this embodiment is a tubular member having an opening into which the proximal end 71b of the distal tube portion 71 is inserted and an opening into which the distal end 72a of the proximal tube portion 72 is inserted. The connecting portion 73 has a center line coaxial with those of the distal tube portion 71 and the proximal tube portion 72.

It is preferable that the connecting portion 73 has a short length in the center line direction of the connecting portion 73 because a length of a rigid part of the sheath 7 can be reduced. In addition, if the length of the connecting portion 73 in the center line direction of the connecting portion 73 is long, it is possible to increase a fixing area for the distal tube portion 71 and the proximal tube portion 72. Therefore, it is possible to reliably fix the distal tube portion 71 and the proximal tube portion 72. The length of the connecting portion 73 in the center line direction of the connecting portion 73 is set such that both flexibility required in the sheath 7 and coupling strength between the distal tube portion 71 and the proximal tube portion 72 can be balanced appropriately.

The covering portion 74 is a resin member that covers the distal tube portion 71 across the entire area of the outer circumferential surface of the distal tube portion 71. The covering portion 74 is formed from, for example, a heat-shrinkable tube which, after the strands constituting the distal tube portion 71 are formed in a coil shape, is tightly fitted to the coil-shaped distal tube portion 71.

The covering portion 74 is formed from a resin tube having a thickness equal to or smaller than that of an outer wall part of the connecting portion 73 in a radial direction of the connecting portion 73. For this reason, while the outer circumferential surface of the distal tube portion 71 is covered with the covering portion 74, the outer diameter of the covering portion 74 is substantially equal to or smaller than that of the connecting portion 73. The covering portion 74 is preferably formed of a material with little influence on the flexibility of the sheath 7. Preferably, the covering portion 74 is smoothly expandable and contractible depending on the bending deformation of the distal tube portion 71 in the sheath 7.

The inner surface of the covering portion 74 is shaped to follow the outer surface shape of the strands constituting the distal tube portion 71. According to this embodiment, the inner surface of the covering portion 74 does not enter a gap between the neighboring strands formed by winding the strands constituting the distal tube portion 71 in a coil shape.

The inner surface of the covering portion 74 is not fixed to the distal tube portion 71. That is, the covering portion 74 is fitted to the outer surface of the distal tube portion 71 by virtue of its own contracting force for reducing the outer diameter of the covering portion 74 to be smaller than the outer diameter of the distal tube portion 71. Therefore, part of the inner surface of the covering portion 74 can be separated from the outer surface of the strands constituting the distal tube portion 71 to allow expansion or contraction when the distal tube portion 71 is bent.

The outer surface of the covering portion 74 has an uneven shape matching the outer surface shape of the strands constituting the distal tube portion 71. Note that the outer surface shape of the covering portion 74 is not particularly limited.

As illustrated in Figs. 5 and 6, the manipulation portion 8 has a manipulation body 9, a sheath adjuster 18 provided at the distal end side of the manipulation body 9, and a needle slider 23 provided at the proximal end side of the manipulation body 9.

The manipulation body 9 is formed of, for example, acrylonitrile butadiene styrene (ABS) resin. The manipulation body 9 has a lumen into which the needle tube 3 and the sheath 7 can be inserted. The distal end side of the manipulation body 9 is inserted into the sheath adjuster 18 formed in a tubular shape. The proximal end side of the manipulation body 9 is inserted into the needle slider 23 formed in a tubular shape. Since grooves or protrusions (not shown) formed on the outer circumferential surface are engaged with each other between the manipulation body 9 and the sheath adjuster 18 and between the manipulation body 9 and the needle slider 23, they can slide along the axial line while their relative rotation around the axial line is suppressed.

A slide lock 51 detachably attached to the proximal end port 108 of the ultrasonic endoscope 100 is provided at the distal end portion of the sheath adjuster 18. By sliding the slide lock 51 perpendicularly to the axial line of the manipulation portion 8 and engaging the slide lock 51 with the proximal end port 108, it is possible to fix the manipulation portion 8 to the ultrasonic endoscope 100. A holder (fixing portion) 52 having a pair of wall portions 52a and 52b is provided at the distal end side of the slide lock 51. The holder 52 is fixed to the sheath adjuster 18. The pair of wall portions 52a and 52b of the holder 52 are arranged substantially in parallel with each other, and a distance therebetween is set such that the distal end side of the manipulation unit 109 of the ultrasonic endoscope 100 can be fitted without rattling.

A support pipe 53 formed of, for example, stainless steel protrudes from the distal end portion of the sheath adjuster 18. The distal end portion of the support pipe 53 is inserted into the channel 107 when the treatment tool 1 is attached to the ultrasonic endoscope 100. The support pipe 53 is inserted into the inside of the manipulation body 9. The proximal end of the support pipe 53 is positioned at the proximal end side relative to the distal end of the needle slider 23 (for example, the position P1 of Fig. 6) when the needle slider 23 fully retracts with respect to the manipulation body 9. The
sheath 7 is inserted into the support pipe 53, and its proximal end portion protrudes from the proximal end of the support pipe 53 and is fixed to the manipulation body 9 using an adhesive and the like.

A fixing screw 54 is attached to the sheath adjuster 18. The fixing screw 54 penetrates the sheath adjuster 18 and is engaged with a screw hole (not shown) provided in the manipulation body 9. If the fixing screw 54 is fastened to the manipulation body 9, the sheath adjuster 18 presses the manipulation body 9, so that the sheath adjuster 18 and the manipulation body 9 can be fixed not to slide. By changing a positional relationship between the sheath adjuster 18 and the manipulation body 9, it is possible to adjust a protrusion length of the sheath 7 from the channel 107 when the manipulation portion 8 is fixed to the ultrasonic endoscope 100. Therefore, it is possible to fix the protrusion length using the fixing screw 54.

As illustrated in Fig. 1, the axial line of the fixing screw 54 is preferably arranged to be directed to the axial line of the manipulation unit 109 fitted in the holder 52. As a result, when the manipulation portion 8 is positioned to face the front, the fixing screw 54 does not deviate to the left or right. Therefore, it is possible to easily perform manipulation regardless of an operator's handedness. If the axial line of the fixing screw 54 is directed to the axial line of the manipulation unit 109 fitted in the holder 52, the similar effect can be obtained even when the fixing screw 54 is attached to the opposite side to that of Fig. 1.

Unevenness is formed on the outer circumferential surface of the distal end portion of the sheath adjuster 18 so that an operator easily holds the sheath adjuster 18.

The needle slider 23 is fixed to the proximal end of the needle tube 3. In addition, the needle slider 23 is connected to the manipulation body 9 so as to be movable with respect to the manipulation body 9. Since the proximal end side of the needle tube 3 protrudes from the proximal end of the sheath 7 and is fixed to the needle slider 23, it is possible to project the needle tube 3 from or retract the needle tube 3 into the distal end of the sheath 7 by sliding the needle slider 23 with respect to the manipulation body 9. In the distal end side of the needle slider 23, a stopper 61 is attached movably with respect to the manipulation body 9. The stopper 61 has a fixing screw 62 and can be fixed to the manipulation body 9 by fastening the fixing screw 62. As illustrated in Fig. 1, the axial line of the fixing screw 62 is preferably arranged to be directed to the axial line of the manipulation unit 109 fitted in the holder 52. As a result, the fixing screw 62 does not deviate to the left or right when the manipulation portion 8 is positioned to face the front. Therefore, it is possible to easily perform manipulation regardless of handedness of an operator. If the axial line of the fixing screw 62 is directed to the axial line of the manipulation unit 109 fitted in the holder 52, the similar effect can be obtained even when the fixing screw 62 is attached in the opposite side to that of Fig. 1.

The fixing screw 62 may be directed either in the same direction as the fixing screw 54 described above or the opposite direction.

As illustrated in Fig. 5, the needle slider 23 is not allowed to advance to the manipulation body 9 out of a contact position with the stopper 61. Therefore, by adjusting the fixing position of the stopper 61 with respect to the manipulation body 9, it is possible to adjust the maximum protrusion length from the sheath 7 of the needle tube 3. According to this embodiment, a manipulation stroke length L2 of the needle tube 3 using the needle slider 23 is set to at least 40 mm.

A state in which the needle slider 23 is located at a position at which the needle slider 23 slides toward the proximal end side of the manipulation body 9 at maximum is an initial state of the treatment tool 1 before stating use. In the initial state, the distal end of the needle tube 3 is placed inside the sheath 7. More specifically, in the initial sate, the distal end of the needle tube 3 is placed inside the distal tube portion 71. In addition, in the positional relationship in which the sheath 7 is attached to the channel 107 of the ultrasonic endoscope 100, and the distal end portion of the sheath 7 can be optically observed using the ultrasonic endoscope 100, the distal end of the needle tube 3 is positioned at the distal end side relative to the distal end 105a of the bending section 105.

According to this embodiment, in the initial state, the needle tube 3, the distal tube portion 71, and the covering portion 74 are disposed inside the channel tube 107c across the entire length of the bending section 105 from the distal end 105a to the proximal end 105b. For this reason, those inserted into the channel tube 107c are constantly stiff across the entire length of the bending section 105, and there is no stiffness difference that may partially change bendability of the bending section 105.

In the initial state, a position of the distal end of the needle tube 3 with respect to the sheath 7 varies depending on influence from expansion or contraction of the sheath 7 and expansion or contraction of the needle tube 3. The variation of the position of the distal end of the needle tube 3 with respect to the sheath 7 is influenced by temperature, humidity, an attachment state of the ultrasonic endoscope 100 to the channel 107, a manipulation force exerted on the treatment tool 1, and the like.

For example, if the distal tube portion 71 and the needle tube 3 are formed of different materials, an expansion coefficient depending on temperature change is different between the distal tube portion 71 and the needle tube 3. Therefore, even in the initial state in which the needle slider 23 is located at a position at which the needle slider 23 slides toward the proximal end side of the manipulation body 9 at maximum, a position of the needle tube 3 with respect to the sheath 7 is different depending on temperature.

In the course of inserting the insertion body 2 into the channel 107 (refer to Fig. 4), the proximal tube portion 72 may receive a contracting force in its center line direction and meander with respect to the center line of the needle tube 3. In this case, when the distal end of the insertion body 2 is guided to the distal end of the channel 107, the distal end of the needle tube 3 is positioned closer to the distal end of the sheath 7 compared to a state in which the insertion body 2 is not inserted into the channel 107.

According to this embodiment, considering temperature, humidity, an attachment state of the ultrasonic endoscope 100 to the channel 107, and a manipulation force exerted on the treatment tool 1, the distal end of the needle tube 3 is set to be positioned inside the distal tube portion 71 at all times in the initial state under an assumed environment as procedures using the treatment tool 1 as illustrated in Fig. 5.

A displacement amount of the needle slider 23 with respect to the manipulation body 9 substantially matches a displacement amount of the distal end of the needle tube 3 with respect to the sheath 7 (refer to Fig. 5). That is, by moving the needle tube 3 with respect to the sheath 7 using the needle slider 23, a displacement amount (relative stroke length L1) of the distal end of the needle tube 3 with respect to the sheath 7 becomes an amount obtained by adding an actual displacement amount of the needle slider 23 (manipulation stroke length L2) to an amount corresponding to expansion or contraction of the needle tube 3. The expansion or contraction of the needle tube 3 is influenced by stretch properties (elasticity) of the needle tube 3, frictional resistance between the needle tube 3 and the sheath 7, a meandering state of the sheath 7 in the channel 107, and a meandering state of the needle tube 3 in the sheath 7.

When the needle slider 23 is located at a position at which the needle slider 23 moves to the distal end side of the manipulation body 9 at maximum, the distal end of the needle tube 3 protrudes from the distal end of the sheath 7. When the needle slider 23 is located at the position at which the needle slider 23 moves to the distal end side of the manipulation body 9 at maximum, the protrusion length of the needle tube 3 is shorter than the manipulation stroke length L2 of the needle slider 23. However, the protrusion length is preferably maintained at at least 40 mm.

An opening 23a is provided in the proximal end portion of the needle slider 23 so that the stylet 27 can be inserted into the needle tube 3 from the proximal end of the needle tube 3. The opening 23a is threaded so that a syringe or the like known in the art can be connected to the opening 23a. Unevenness is formed on the outer circumferential surface of the distal end portion of the needle slider 23 so that an operator easily holds the needle slider 23.

The stylet 27 illustrated in Figs. 3 and 5 has a knob 27a that can be attached to the opening 23a of the needle slider 23 and a core 27b fixed to the knob 27a. The core 27b has a cross-sectional shape matching the shape of the inner surface of the needle tube 3. According to this embodiment, the core 27b has a circular cross section.

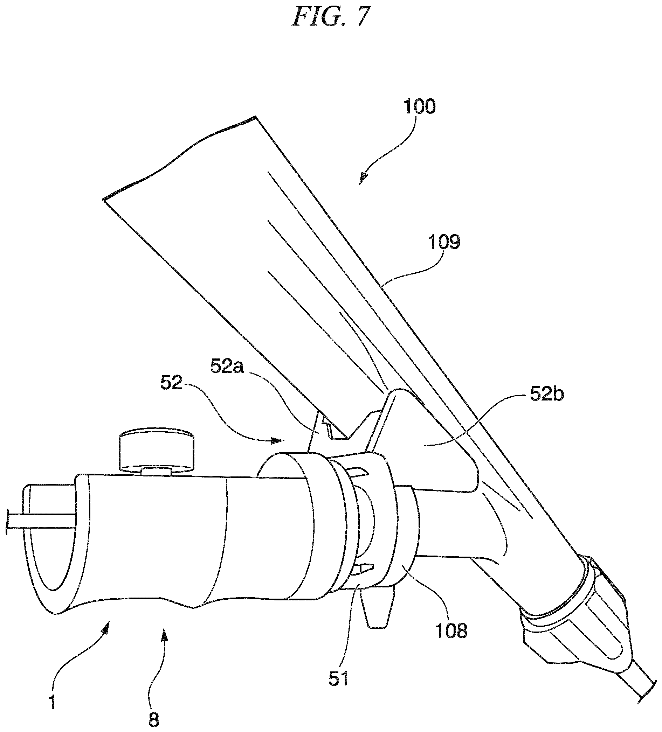

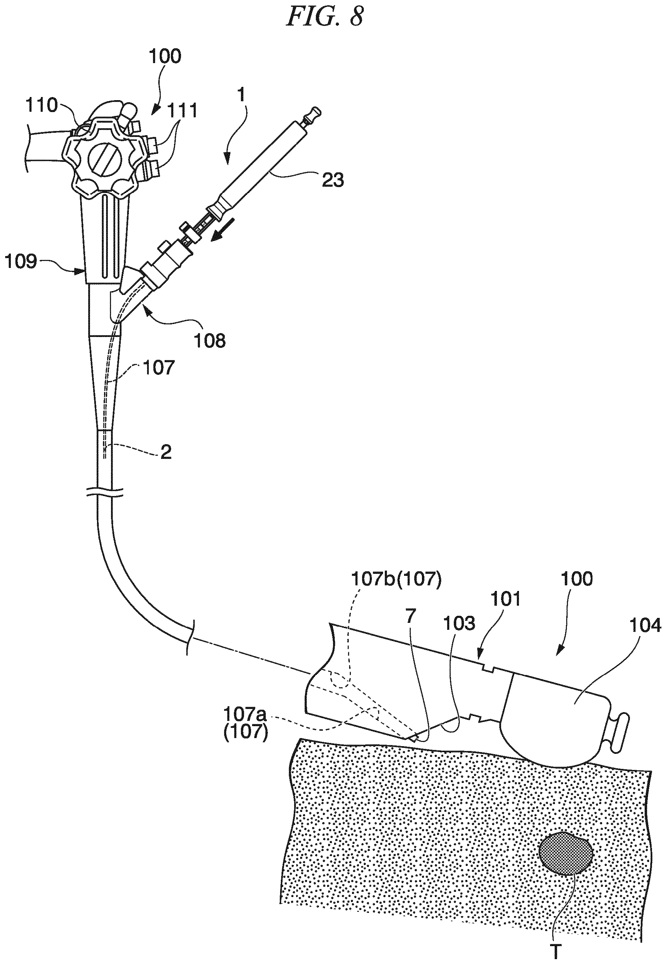

Operations in use of the biopsy system 150 having the aforementioned configuration will be described. Fig. 7 is a perspective view illustrating an attachment state between the treatment tool 1 and the ultrasonic endoscope 100. Figs. 8 to 10 are schematic diagrams for describing operations of the biopsy system 150. Hereinafter, an exemplary biopsy treatment in which the needle tube 3 of the treatment tool 1 is inserted into a lesion as a target tissue positioned in a deep part of a lung to capture cells and the like of the lesion through the inside of the needle tube 3 will be described.

First, an operator inserts the insertion portion 101 of the ultrasonic endoscope 100 illustrated in Fig. 1 into a living body and introduces the distal end of the insertion portion 101 to the vicinity of the target tissue by appropriately bending the bending section 105 while monitoring the status using the optical imaging mechanism 103. After introduction, the operator determines a biopsy target on the basis of a result of the monitoring using the optical imaging mechanism 103 and the ultrasonic scanning mechanism 104.

Then, the operator inserts the insertion body 2 of the treatment tool 1 into the channel 107 from the proximal end port 108 provided at the manipulation unit 109 of the ultrasonic endoscope 100 from the distal end side. In addition, the operator inserts the distal end side of the manipulation unit 109 into a gap between the pair of wall portions 52a and 52b of the holder 52 as illustrated in Fig. 7 and engages the slide lock 51 provided in the manipulation portion 8 of the treatment tool 1 with the proximal end port 108. As a result, the manipulation portion 8 of the treatment tool 1 is fixed to the ultrasonic endoscope 100 so that it does not rotate with respect to the manipulation unit 109.

In this case, the treatment tool 1 is placed in the initial state described above, that is, the distal end of the needle tube 3 is placed inside the distal tube portion 71. Since the covering portion 74 is tightly fitted to the distal tube portion 71, the strands constituting the distal tube portion 71 are held in a tightly winding state. As the distal tube portion 71 is deformed to follow the bending shape of the channel 107, part of the strands constituting the distal tube portion 71 are separated from each other, so that the covering portion 74 is stretched to follow the bending deformation of the distal tube portion 71. Deviation of the interval between the strands of the distal tube portion 71 is suppressed because the covering portion 74 is tightly fitted to the strands of the distal tube portion 71.

The needle tube 3 guided inside the channel 107 along the bent flexible tube section 106 is protected such that the distal end of the needle tube 3 does not puncture the distal tube portion 71. In addition, due to frictional resistance between the inner surface of the channel 107 and the outermost surface of the insertion body 2, a contracting or meandering caused by compression of the sheath 7 may be accumulated as the insertion body 2 is pushed into the channel 107. In this case, the distal end of the sheath 7 moves to the proximal end side relative to the needle tube 3. However, even in this case, the distal end of the needle tube 3 is protected by the distal tube portion 71.

Then, the operator loosens the fixing screw 54, slides the sheath adjuster 18 and the manipulation body 9 relatively as illustrated in Fig. 8 while monitoring the sheath 7 and the inside of the living body using the optical imaging mechanism 103 and the ultrasonic scanning mechanism 104, and appropriately adjusts the protrusion length of the sheath 7 from the distal end of the insertion portion 101 of the ultrasonic endoscope 100. After the adjustment, the operator fastens the fixing screw 54 to fix the protrusion length.

Even after the protrusion length of the sheath 7 is adjusted, the distal end of the needle tube 3 still remains inside the distal tube portion 71. In addition, in the channel 107, the distal end of the needle tube 3 is placed in any position between the bending section 105 and the angle tube 107b, inside the angle tube 107b, or inside the slope portion 107a.

A position of the connecting portion 73 in the sheath 7 is closer to the proximal end side relative to the proximal end 105b of the bending section 105 of the ultrasonic endoscope 100. Specifically, when the distal end of the sheath 7 can be optically observed using the ultrasonic endoscope 100, the connecting portion 73 is positioned closer to the proximal side relative to the proximal end 105b of the bending section 105. In other words, when the distal end of the sheath 7 can be optically observed using the ultrasonic endoscope 100, only the distal tube portion 71 and the needle tube 3 are disposed in an area ranging from the proximal end 105b of the bending section 105 to the distal end of the insertion portion 101.

Since only the distal tube portion 71 and the needle tube 3 are disposed in the area ranging from the proximal end 105b of the bending section 105 to the distal end of the insertion portion 101, degradation of the bendability caused by the bending section 105 may not easily occur, compared to a case in which the proximal tube portion 72 is placed inside the bending section 105.

Since the proximal tube portion 72 is disposed in the area closer to the proximal end side relative to the proximal end 105b of the bending section 105, the sheath 7 does not easily meander in this area.

Then, on the basis of the monitoring result using the ultrasonic scanning mechanism 104, the stopper 61 is moved and fixed at a desired position to the manipulation body 9 in consideration of the distance to the target tissue T on which a biopsy is to be performed, and the maximum protrusion length of the needle tube 3 is adjusted.

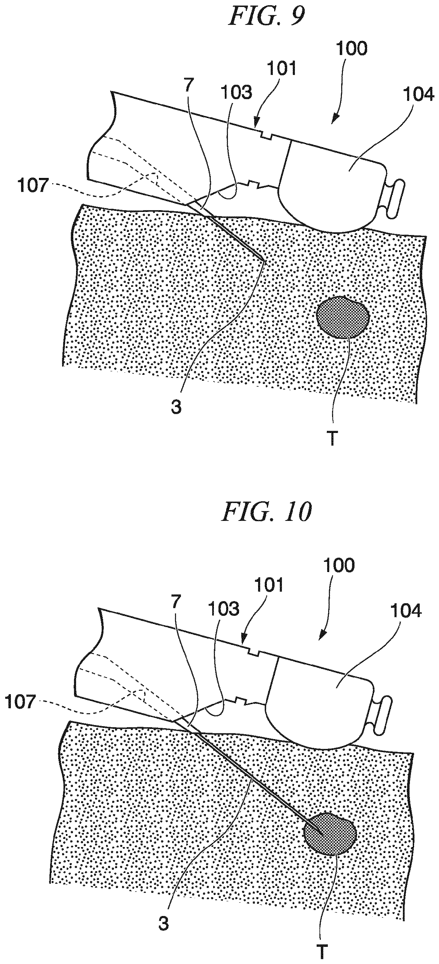

Then, as illustrated in Fig. 8, the operator advances the needle slider 23 toward the distal end side of the manipulation portion 8. As a result, as illustrated in Fig. 9, the needle tube 3 protrudes from the sheath 7. If the operator advances the needle slider 23 to the distal end side of the manipulation portion 8 while the distal end of the needle tube 3 is placed between the bending section 105 and the angle tube 107b, the distal end of the needle tube 3 passes through the angle tube 107b and reaches the slope portion 107a while the distal end of the needle tube 3 is guided by the distal tube portion 71 of the sheath 7.

When the distal end of the needle tube 3 is shifted to the distal end side with the angle tube 107b set as a starting point, and when the distal end of the needle tube 3 is shifted to the distal end side with the slope portion 107a set as a starting point, the distal end of the needle tube 3 protrudes from the distal end of the sheath 7 while it is guided by the distal tube portion 71 as described above.

As the operator advances the needle slider 23 to the distal end side of the manipulation portion 8, the distal end of the needle tube 3 is inserted into tissue and is pushed to advance toward the target tissue T on which the biopsy is to be performed as illustrated in Fig. 10. Then, the needle tube 3 exposed to the outside from the surface of the tissue can be observed using the optical imaging mechanism 103. The distal end side portion of the needle tube 3 inserted into the inside of the tissue can be observed using the ultrasonic scanning mechanism 104.

The operator can observe an ultrasonic image based on an ultrasonic wave received by the ultrasonic scanning mechanism 104 using the ultrasonic monitoring unit 115 illustrated in Fig. 1. The operator monitors the image of the needle tube 3 clearly displayed on the ultrasonic monitoring unit 115, and places the distal end of the needle tube 3 on the target tissue T on which the biopsy is to be performed.

Then, the operator extrudes a non-biopsy target tissue inserted into the needle tube 3 using the stylet 27 and extracts the stylet 27 from the insertion body 2 and the manipulation portion 8. As a result, a through-hole extending from the distal end of the needle tube 3 to the proximal end of the needle slider 23 is formed. The operator connects a syringe or the like to the proximal end of the needle slider 23 and suctions the inside of the needle tube 3 to capture cells or the like of the target tissue T on which the biopsy is to be performed from the distal end of the needle tube 3.

Once cells or the like are captured in a necessary amount, the needle slider 23 retracts to the proximal end side of the manipulation portion 8 to house the distal end of the needle tube 3 inside the sheath 7. As a result, the needle tube 3 is pulled out from the tissue. Once the needle tube 3 is pulled out from the tissue, the slide lock 51 is released from the proximal end port 108 of the manipulation unit 109 of the ultrasonic endoscope 100, and the treatment tool 1 is extracted from the channel 107. Finally, the ultrasonic endoscope 100 is removed from a patient, and a series of treatment procedures is finished.

As described above, according to this embodiment, when the distal end portion of the sheath 7 has a positional relationship optically observable by the ultrasonic endoscope 100, the connecting portion 73 corresponding to a boundary between the distal tube portion 71 and the proximal tube portion 72 is positioned closer to the proximal end side relative to the proximal end of the bending section 105. There is a difference in flexibility between the distal tube portion 71 and the proximal tube portion 72 such that the distal tube portion 71 has flexibility higher than that of the proximal tube portion 72. Therefore, according to this embodiment, while the treatment tool 1 is attached to the ultrasonic endoscope 100, bendability of the bending section 105 does not easily degrade, and expansion/contraction of the sheath 7 inside the channel 107 in the center line direction or meandering of the sheath 7 does not easily occur.

The covering portion 74 arranged to be tightly fitted to the outer surface of the distal tube portion 71 prevents the strands constituting the distal tube portion 71 from being extremely separated during bending deformation of the distal tube portion 71. Therefore, the distal end of the needle tube 3 does not easily enter a gap between the strands, and it is possible to prevent the needle tube 3 from puncturing the sheath 7.

Since the connecting portion 73 is placed closer to the proximal end side relative to the proximal end of the bending section 105, the proximal tube portion 72 does not enter the inside of the bending section 105 even when the position of the sheath 7 is adjusted inside the channel 107. For this reason, bendability of the bending section 105 does not vary before and after the position adjustment.

While preferred embodiments of the invention have been described and illustrated hereinbefore, specific configurations are not limited to the embodiments, and any design change or the like may be possible without departing from the scope of the present invention.

For example, instead of the bent or curved angle tube 107b having a tubular shape, a through-hole (angle portion) bent or curved similarly to the angle tube 107b may be formed in the distal end hard section 102. In this case, the angle tube 107b is not necessary.

Instead of connecting the distal tube portion 71 and the proximal tube portion 72 using a tubular member, the connecting portion 73 may be formed by welding the distal tube portion 71 and the proximal tube portion 72. The connecting portion 73 may be a simple boundary position where a configuration of the strand changes. For example, in a coil obtained by winding the strands continuously from the distal end to the proximal end of the sheath 7, the difference of the flexibility similar to that described in the above embodiment may be introduced by changing a cross-sectional area, a cross-sectional shape, hardness, and the like of the strands in the position of the connecting portion 73.

The treatment tool may not have the covering portion 74.

The invention is not to be considered as being limited by the foregoing description, and is only limited by the appended claims.

### Industrial Applicability

According to the present invention, it is possible to provide a biopsy system capable of suppressing a flexible sheath from easily expanding or contracting as a whole and preventing degradation of bendability of a bending section of the endoscope.

### Reference Signs List

1: treatment tool (endoscopic treatment tool)
2: insertion body
3: needle tube
31: opening of needle tube
7: sheath
71: distal tube portion
71a: distal end of distal tube portion
71b: proximal end of distal tube portion
72: proximal tube portion
72a: distal end of proximal tube portion
73: connecting portion
74: covering portion (cover)
8: manipulation portion
9: manipulation body
18: sheath adjuster
23: needle slider
27: stylet (core metal)
27a: knob
27b: core
51: slide lock
52: holder (fixing portion)
52a, 52b: pair of wall portions
53: support pipe
54: fixing screw
61: stopper
62: fixing screw
100: ultrasonic endoscope
101: insertion portion
102: distal end hard section
103: optical imaging mechanism (imaging unit)
104: ultrasonic scanning mechanism
105: bending section
106: flexible tube section
107: channel
107a: slope portion
107b: angle tube (angle portion)
107c: channel tube
108: proximal end port
109: manipulation unit
110: bending manipulation mechanism
111: switches
112: universal cord
113: light source
114: optical monitoring unit
115: ultrasonic monitoring unit
116: monitor
150: biopsy system

## Claims

1. A biopsy system (150) comprising:
an endoscope (100) having an insertion portion (101) provided with a channel (107) and inserted into a living body, and a bending section (105) disposed in part of the insertion portion (101) and configured to cause part of the channel (107) to bend; and
an endoscopic treatment tool (1) capable of being inserted into the channel (107),
wherein the endoscope (100) further comprises an imaging unit (103) capable of imaging the endoscopic treatment tool (1) and a biopsy target portion,
wherein the endoscopic treatment tool (1) has:
a tubular flexible sheath (7) capable of being inserted into the channel (107);
a needle tube (3) provided with a sharp end portion capable of puncturing a tissue for biopsy and disposed inside the sheath (7); and
a manipulation portion (8) having a needle slider (23) connected to a proximal end of the needle tube (3) and a manipulation body (9) connected to a proximal end of the sheath (7), the manipulation portion (8) being configured to move the needle tube (3) inside the sheath (7) to cause the needle tube (3) to protrude from and retract into a distal end of the sheath (7) by movement of the needle slider (23) relative to the manipulation body (9), and
wherein the sheath (7) has:
a distal tube portion (71) disposed in a distal portion of the sheath (7) including the distal end of the sheath (7);
a proximal tube portion (72) fixed to a proximal end of the distal tube portion (71) and aligned along the same axial line as that of the distal tube portion (71), the proximal tube portion (72) having flexibility lower than that of the distal tube portion (71); and
a connecting portion (73), which is configured to connect the distal tube portion (71) and the proximal tube portion (72), and which is positioned in a proximal side relative to a proximal end of the bending section (105) in a positional relationship in which the distal end of the sheath (7) protrudes from a distal end of the channel (107), and is optically observable by the endoscope.

2. The biopsy system (150) according to claim 1, wherein
the connecting portion (73) is a tubular member in which the proximal end (71b) of the distal tube portion (71) and a distal end (72a) of the proximal tube portion (72) are internally placed together,
the distal tube portion (71) has:
a flexible coil fixed to the connecting portion (73); and
a cover (74) attached to the coil such that the cover (74) covers an outer circumferential surface of the coil, and
a difference between an outer diameter of the cover (74) and an outer diameter of the connecting portion (73) is smaller than a difference between an outer diameter of the proximal tube portion (72) and the outer diameter of the connecting portion (73).

3. The biopsy system (150) according to claim 2, wherein an elastic deformation amount of the distal tube portion (71) per unit length in a center line direction caused by a self weight of the distal tube portion (71) is larger than an elastic deformation amount of the proximal tube portion (72) per unit length in the center line direction caused by the self weight of the proximal tube portion (72).

4. The biopsy system (150) according to claim 3, wherein an outer circumferential surface of the cover (74) has an uneven shape to follow the outer circumferential surface of the coil.

5. The biopsy system (150) according to claim 4, wherein
the endoscope (100) has:
the imaging unit (103) capable of imaging the endoscopic treatment tool (1) and a biopsy target portion; and
an ultrasonic scanning mechanism (104) disposed in a distal side relative to the imaging unit (103),
the channel (107) has:
a slope portion (107a) sloped with respect to a center line of the insertion portion (101) such that sheath (7) is bent toward a scanning range of the ultrasonic scanning mechanism (104);
a channel tube (107c) arranged in parallel with the center line of the insertion portion (101); and
an angle portion (107b) configured to link the slope portion (107a) and the channel tube (107c), and
in the positional relationship in which the distal end of the sheath (7) protrudes from the distal end of the channel (107) and is optically observable by the endoscope (100), the end portion of the needle tube (3) is placed in a distal end side relative to the bending section (105) and in a proximal side relative to the slope portion (107a) when the end portion of the needle tube (3) is maximally shifted to the proximal side by the manipulation portion (8).

## Patentansprüche

1. Biopsiesystem (150), umfassend:
ein Endoskop (100), das einen Einführabschnitt (101), der mit einem Kanal (107) versehen ist und in einen lebenden Körper eingeführt wird, und einen Biegeabschnitt (105) aufweist, der in einem Teil des Einführabschnitts (101) angeordnet und so konfiguriert ist, dass er bewirkt, dass sich ein Teil des Kanals (107) biegt; und
ein endoskopisches Behandlungsinstrument (1), das dazu eingerichtet ist, in den Kanal eingeführt (107) zu werden,
wobei das Endoskop (100) ferner eine Bildgebungseinheit (103) umfasst, die dazu eingerichtet ist, das endoskopische Behandlungsinstrument (1) und einen Biopsie-Zielabschnitt abzubilden,
wobei das endoskopische Behandlungsinstrument (1) aufweist:
eine röhrenförmige flexible Hülse (7), die dazu eingerichtet ist, in den Kanal eingeführt (107) zu werden;
eine Nadelröhre (3), die mit einem scharfen Endabschnitt versehen ist, der dazu eingerichtet ist, ein Gewebe zur Biopsie zu punktieren und in der Hülse (7) angeordnet ist; und
einen Handhabungsabschnitt (8), der einen Nadelschieber (23), der mit einem proximalen Ende der Nadelröhre (3) verbunden ist, und einen Handhabungskörper (9) aufweist, der mit einem proximalen Ende der Gülse (7) verbunden ist, wobei der Handhabungsabschnitt (8) zum Bewegen der Nadelröhre (3) in der Hülse (7) konfiguriert ist, um zu bewirken, dass die Nadelröhre (3) durch Bewegung des Nadelschiebers (23) bezüglich des Handhabungskörpers (9) aus dem distalen Ende der Hülse (7) hervorsteht oder in dieses zurückgezogen wird, und
wobei die Hülse (7) aufweist:
einen distalen Röhrenabschnitt (71), der in einem distalen Abschnitt der Hülse (7) angeordnet ist, der das distale Ende der Hülse (7) umfasst;
einen proximalen Röhrenabschnitt (72), der an einem proximalen Ende des distalen Röhrenabschnitts (71) befestigt und entlang der gleichen Achsenlinie wie der distale Röhrenabschnitt (71) ausgerichtet ist, wobei der proximale Röhrenabschnitt (72) eine geringere Flexibilität als der distale Röhrenabschnitt (71) aufweist; und
einen Verbindungsabschnitt (73), der zum Verbinden des distalen Röhrenabschnitts (71) und des proximalen Röhrenabschnitts (72) konfiguriert ist, und der auf einer proximalen Seite bezüglich eines proximalen Endes des Biegeabschnitts (105) in einem Positionsverhältnis angeordnet ist, in dem das distale Ende der Hülse (7) aus einem distalen Ende des Kanals (107) hervorsteht und vom Endoskop optisch beobachtbar ist.

2. Biopsiesystem (150) nach Anspruch 1, wobei
der Verbindungsabschnitt (73) ein röhrenförmiges Glied ist, in dem das proximale Ende (71b) des distalen Röhrenabschnitts (71) und ein distales Ende (72a) des proximalen Röhrenabschnitts (72) intern zusammen angeordnet sind,
der distale Röhrenabschnitt (71) aufweist:
eine flexible Spule, die am Verbindungsabschnitt (73) befestigt ist; und
eine Abdeckung (74), die so an der Spule befestigt ist, dass die Abdeckung (74) eine äußere Umfangsfläche der Spule abdeckt, und
eine Differenz zwischen einem Außendurchmesser der Abdeckung (74) und einem Außendurchmesser des Verbindungsabschnitts (73) kleiner als eine Differenz zwischen einem Außendurchmesser des proximalen Röhrenabschnitts (72) und dem Außendurchmesser des Verbindungsabschnitts (73) ist.

3. Biopsiesystem (150) nach Anspruch 2, wobei ein Betrag einer elastischen Verformung des distalen Röhrenabschnitts (71) pro Einheitslänge in Richtung einer Mittellinie, die von einem Eigengewicht des distalen Röhrenabschnitts (71) verursacht wird, größer als ein Betrag einer elastischen Verformung des proximalen Röhrenabschnitts (72) pro Einheitslänge in Richtung der Mittellinie ist, die vom Eigengewicht des proximalen Röhrenabschnitts (72) verursacht wird.

4. Biopsiesystem (150) nach Anspruch 3, wobei eine äußere Umfangsfläche der Abdeckung (74) eine unregelmäßige Form aufweist, um der äußeren Umfangsfläche der Spule zu folgen.

5. Biopsiesystem (150) nach Anspruch 4, wobei
das Endoskop (100) aufweist:
die Bildgebungseinheit (103), die dazu eingerichtet ist, das endoskopische Behandlungsinstrument (1) und einen Biopsie-Zielabschnitt abzubilden; und
einen Ultraschall-Abtastmechanismus (104), der auf einer distalen Seite bezüglich der Bildgebungseinheit (103) angeordnet ist,
der Kanal (107) aufweist:
einen Neigungsabschnitt (107), der bezüglich einer Mittellinie des Einführabschnitts (101) so geneigt ist, dass die Hülse (7) zu einem Abtastbereich des Ultraschall-Abtastmechanismus (104) gebogen ist;
eine Kanalröhre (107c), die parallel zur Mittellinie des Einführabschnitts (101) angeordnet ist; und
einen Winkelabschnitt (107b), der zum Verbinden des Neigungsabschnitts (107a) und der Kanalröhre (107c) konfiguriert ist; und
im Positionsverhältnis, in dem das distale Ende der Hülse (7) vom distalen Ende des Kanals (107) hervorsteht und vom Endoskop (100) optisch beobachtbar ist, der Endabschnitt der Nadelröhre (3) auf einer distalen Endseite bezüglich des Biegeabschnitts (105) und auf einer proximalen Seite bezüglich des Neigungsabschnitts (107a) platziert ist, wenn der Endabschnitt der Nadelröhre (3) vom Handhabungsabschnitt (8) maximal zur proximalen Seite verschoben ist.

## Revendications

1. Système de biopsie (150) comprenant :
un endoscope (100) ayant une partie d'insertion (101) comportant un canal (107) et insérée dans un corps vivant, et une section de courbure (105) disposée dans une section de la partie d'insertion (101) et configurée pour amener une partie du canal (107) à se courber ; et
un outil de traitement endoscopique (1) capable d'être inséré dans le canal (107),
l'endoscope (100) comprenant en outre une unité d'imagerie (103) capable d'imager l'outil de traitement endoscopique (1) et une partie cible de biopsie,
l'outil de traitement endoscopique (1) ayant :
une gaine souple tubulaire (7) capable d'être insérée dans le canal (107) ;
un tube d'aiguille (3) comportant une partie d'extrémité tranchante capable de perforer un tissu pour une biopsie et disposé à l'intérieur de la gaine (7) ; et
une partie de manipulation (8) ayant un coulisseau d'aiguille (23) relié à une extrémité proximale du tube d'aiguille (3) et un corps de manipulation (9) relié à une extrémité proximale de la gaine (7), la partie de manipulation (8) étant configurée pour déplacer le tube d'aiguille (3) à l'intérieur de la gaine (7) afin d'amener le tube d'aiguille (3) à faire saillie à partir de et se rétracter dans une extrémité distale de la gaine (7) par mouvement du coulisseau d'aiguille (23) par rapport au corps de manipulation (9), et
la gaine (7) ayant :
une partie de tube distale (71) disposée dans une partie distale de la gaine (7) comprenant l'extrémité distale de la gaine (7) ;
une partie de tube proximale (72) fixée à une extrémité proximale de la partie de tube distale (71) et alignée le long de la même ligne axiale que celle de la partie de tube distale (71), la partie de tube proximale (72) ayant une flexibilité inférieure à celle de la partie de tube distale (71) ; et
une partie de liaison (73), qui est configurée pour relier la partie de tube distale (71) et la partie de tube proximale (72), et qui est positionnée sur un côté proximal par rapport à une extrémité proximale de la section de courbure (105) dans une relation de position dans laquelle l'extrémité distale de la gaine (7) fait saillie à partir d'une extrémité distale du canal (107), et peut être observée optiquement par l'endoscope.

2. Système de biopsie (150) selon la revendication 1, dans lequel
la partie de liaison (73) est un élément tubulaire dans lequel l'extrémité proximale (71b) de la partie de tube distale (71) et une extrémité distale (72a) de la partie de tube proximale (72) sont placées ensemble à l'intérieur,
la partie de tube distale (71) a :
une bobine souple fixée à la partie de liaison (73) ; et
un revêtement (74) fixé à la bobine de telle sorte que le revêtement (74) recouvre une surface circonférentielle externe de la bobine, et
une différence entre un diamètre externe du revêtement (74) et un diamètre externe de la partie de liaison (73) est plus petite qu'une différence entre un diamètre externe de la partie de tube proximale (72) et le diamètre externe de la partie de liaison (73).

3. Système de biopsie (150) selon la revendication 2, dans lequel une quantité de déformation élastique de la partie de tube distale (71) par unité de longueur dans une direction de ligne centrale causée par le propre poids de la partie de tube distale (71) est plus grande qu'une quantité de déformation élastique de la partie de tube proximale (72) par unité de longueur dans la direction de ligne centrale causée par le propre poids de la partie de tube proximale (72).

4. Système de biopsie (150) selon la revendication 3, dans lequel une surface circonférentielle externe du revêtement (74) a une forme irrégulière pour suivre la surface circonférentielle externe de la bobine.

5. Système de biopsie (150) selon la revendication 4, dans lequel l'endoscope (100) a :
l'unité d'imagerie (103) capable d'imager l'outil de traitement endoscopique (1) et une partie cible de biopsie ; et
un mécanisme de balayage par ultrasons (104) disposé sur un côté distal par rapport à l'unité d'imagerie (103),
le canal (107) a :
une partie d'inclinaison (107a) inclinée par rapport à une ligne centrale de la partie d'insertion (101) de telle sorte que la gaine (7) est courbée vers une plage de balayage du mécanisme de balayage par ultrasons (104) ;
un tube de canal (107c) disposé en parallèle avec la ligne centrale de la partie d'insertion (101) ; et
une partie d'angle (107b) configurée pour relier la partie d'inclinaison (107a) et le tube de canal (107c), et
dans la relation de position dans laquelle l'extrémité distale de la gaine (7) fait saillie à partir de l'extrémité distale du canal (107) et peut être observée optiquement par l'endoscope (100), la partie d'extrémité du tube d'aiguille (3) est placée sur un côté extrémité distale par rapport à la section de courbure (105) et sur un côté proximal par rapport à la partie d'inclinaison (107a) lorsque la partie d'extrémité du tube d'aiguille (3) est décalée au maximum vers le côté proximal par la partie de manipulation (8).
